# EUROPEAN PATENT SPECIFICATION

(11) **EP 2 423 232 B1**
(45) Date of publication and mention of the grant of the patent: **21.05.2014**
(21) Application number: 10766905.3
(22) Date of filing: 08.03.2010
(51) Int. Cl.: C07K 16/30, G01N 33/574, C07K 16/18, C07K 16/40

(54) **COCKTAIL ANTIBODY FOR DETERMINING HISTOLOGICAL TYPE OF CARCINOMA, DETERMINATION KIT AND DETERMINATION METHOD**
COCKTAIL-ANTIKÖRPER ZUR BESTIMMUNG DES HISTOLOGISCHEN TYPS EINES KARZINOMS SOWIE BESTIMMUNGSKIT UND BESTIMMUNGSVERFAHREN
ANTICORPS EN COCKTAIL POUR DÉTERMINER UN TYPE HISTOLOGIQUE DE CARCINOME, ENSEMBLE ET PROCÉDÉ DE DÉTERMINATION

(30) Priority: 20.04.2009 JP 2009102161
(43) Date of publication of application: 29.02.2012
(73) Proprietor: National University Corporation University Of Toyama, Toyama-shi, Toyama 930-8555 (JP)
(72) Inventor: FUKUOKA, Jyunya, Toyama-shi Toyama 930-0194 (JP); TANI, Yoichi, Toyama-shi Toyama 930-0871 (JP)
(74) Representative: UEXKÜLL & STOLBERG
(86) International application number: PCT/JP2010/053759
(87) International publication number: WO 2010/122846

(56) References cited:
- TSUTA KOHJI: "Neuroendocrine tumor <Special Articles> Small cell tumor and differential diagnosis [In Japanese]", THE JOURNAL OF THE JAPANESE SOCIETY OF CLINICAL CYTOLOGY, vol. 47, no. 2, 2008, pages 146-147, XP002683255, Japanese Society of Clinical Cytology ISSN: 0387-1193
- FUTOSHI TSUBOKAWA ET AL: "Heterogeneity of expression of cytokeratin subtypes in squamous cell carcinoma of the lung: With special reference to CK14 overexpression in cancer of high-proliferative and lymphogenous metastatic potential", PATHOLOGY INTERNATIONAL, vol. 52, no. 4, 1 April 2002 (2002-04-01), pages 286-293, XP55037148, ISSN: 1320-5463, DOI: 10.1046/j.1440-1827.2002.01353.x
- SU Y C ET AL: "Role Of TTF-1, CK20, And CK7 Immunohistochemistry for Diagnosis of Primary and Secondary Lung Adenocarcinoma", KAOHSIUNG JOURNAL OF MEDICAL SCIENCES, KAOHSIUNG MEDICAL COLLEGE, KAOHSIUNG, TW, vol. 22, no. 1, 1 January 2006 (2006-01-01), pages 14-19, XP026304992, ISSN: 1607-551X, DOI: 10.1016/S1607-551X(09)70214-1 [retrieved on 2006-01-01]
- DE PERALTA-VENTURINA M ET AL: "Clinical Utility of Immunohistochemistry for the Diagnosis of Lung Squamous Cell Carcinoma in Biopsy and Cytologic Specimens", MODERN PATHOLOGY, NATURE PUBLISHING GROUP, US, vol. 23, no. supplement 1, 1 February 2010 (2010-02-01), pages 400A-401A, XP008140459, ISSN: 0893-3952, DOI: 10.1038/MODPATHOL.2010.27
- UENO T ET AL: "Aspartic proteinase napsin is a useful marker for diagnosis of primary lung adenocarcinoma", BRITISH JOURNAL OF CANCER, HARCOURT PUBLISHERS, vol. 88, no. 8, 22 April 2003 (2003-04-22) , pages 1229-1233, XP002359371, ISSN: 0007-0920, DOI: 10.1038/SJ.BJC.6600879
- TSUBOKAWA F. ET AL.: 'Heterogeneity of expression of cytokeratin subtypes in squamous cell carcinoma of the lung: with special reference to CK14 overexpression in cancer of high- proliferative and lymphogenous metastatic potential.' PATHOL. INT. vol. 52, no. 4, 2002, pages 286 - 293, XP055037148
- KARGI A. ET AL.: 'The diagnostic value of TTF-1, CK 5/6, and p63 immunostaining in classification of lung carcinomas.' APPL. IMMUNOHISTOCHEM. MOL. MORPHOL. vol. 15, no. 4, 2007, pages 415 - 420, XP009162652
- N Sturm ET AL: "34betaE12 expression along the whole spectrum of neuroendocrine proliferations of the lung, from neuroendocrine cell hyperplasia to small cell carcinoma", Histopathology, vol. 42, no. 2, 1 February 2003 (2003-02-01), pages 156-166, XP055064262, ISSN: 0309-0167, DOI: 10.1046/j.1365-2559.2003.01541.x

## Description

### TECHNICAL FIELD

The present invention relates to a method that immunohistochemically determines the histological type of cancer. More particularly, the invention relates to a cocktail antibody that is effective for determining the histological type of non-small cell lung carcinoma, a method of determining the histological type of non-small cell lung carcinoma using the cocktail antibody, and a kit for determining the histological type of non-small cell lung carcinoma.

### BACKGROUND ART

It is estimated that a million or more people die of lung cancer each year in the world. Lung carcinoma is histologically classified as small cell lung carcinoma (SCLC) or non-small cell lung carcinoma (NSCLC). Non-small cell lung carcinoma is classified as adenocarcinoma (ADC), squamous cell carcinoma (SCC), or large cell carcinoma which is undifferentiated non-small cell lung carcinoma.

It was confirmed with reproducibility that pemetrexed and gefitinib used to treat lung carcinoma have a different effect depending on the histological type.

In that study, it was shown that it is especially important to distinguish squamous cell carcinoma and adenocarcinoma.

It was also demonstrated that the prognosis of patients who develop squamous cell carcinoma and were treated with pemetrexed was poor as compared with a placebo.

In view of the above findings, the necessity of a method that conveniently determines (distinguishes) squamous cell carcinoma and adenocarcinoma with high sensitivity and high specificity has been discussed in international conference.

Non-patent Document 1 discloses a method for immunohistochemical determination of lung carcinoma, comprising: a first step that determines (distinguishes) a high-grade neuroendocrine tumor, adenocarcinoma, and squamous cell carcinoma using thyroid transcription factor 1 (TTF-1) which is a transcription factor specifically expressed in a thyroid gland and lungs, Napsin-A which is a specific marker of primary lung adenocarcinoma, and p63 which is tumor suppressor protein; and a second step that determines (distinguishes) large cell neuroendocrine carcinoma (LCNEC), as a high-grade neuroendocrine tumor, and small cell carcinoma by using cytokeratin 18 (CK 18).

Keratin is an epithelial cytoskeletal protein, and about 20 subtypes of the keratin have been known.

For example, the staining characteristics of cytokeratin 7 (CK7) and cytokeratin 20 (CK20) is utilized to determine tumor cells.

However, since CK18 is positive for adenocarcinoma and squamous cell carcinoma, it is not suitable as a marker of squamous cell carcinoma.

Moreover, the positive reactivity of p63 in squamous cell carcinoma is not 100%.

A method that conveniently determines (distinguishes) squamous cell carcinoma and adenocarcinoma with high sensitivity and high specificity has been desired.
Non-patent Document 1; TSUTA KOHJI, Neuroendocrine tumor (Special Articles) Small cell tumor and differential diagnosis, The Journal of the Japanese Society of Clinical Cytology, Vol. 47 No. 2, pp. 146-147 (2008).

Further prior art is disclosed in:
FUTOSHI TSUBOKAWA ET AL: "Heterogeneity of expression of cytokeratin subtypes in squamous cell carcinoma of the lung: With special reference to CK14 overexpression in cancer of high-proliferative and lymphogenous metastatic potential", PATHOLOGY INTERNATIONAL, vol. 52, no. 4, April 2002, pages 286-293.
SU Y C ET AL: "Role of TTF-1, CK20, and CK7. Immunohistochemistry for Diagnosis of Primary and Secondary Lung Adenocarcinoma", KAOHSIUNG JOURNAL OF MEDICAL SCIENCES, KAOHSIUNG MEDICAL COLLEGE, KAOHSIUNG, TW, vol. 22, no. 1, January 2006 pages 14-19.
KARGI A. ET AL.: "The diagnostic value of TTF-1, CK 5/6, and p63 immunostaining in classification of lung carcinomas.", APPL. IMMUNOHISTOCHEM. MOL. MORPHOL, vol. 15, no. 4, 2007, pages 415-420.
N Sturm ET AL: "34βE12 expression along the whole spectrum of neuroendocrine proliferations of the lung, from neuroendocrine cell hyperplasia to small cell carcinoma", Histopathology, vol. 42, no. 2, February 2003, pages 156-166.

### DISCLOSURE OF THE INVENTION

### PROBLEMS TO BE SOLVED BY THE INVENTION

An object of the invention is to provide a cocktail antibody useful for determining the histological type of cancer, and a determination method using the same cocktail antibody.

Another object of the invention is to provide a determination kit.

### MEANS FOR SOLVING THE PROBLEMS

A cocktail antibody of the present invention is a cocktail antibody used to determine the histological type of cancer. The cocktail antibody comprises a combination of an antibody that shows an antigen-antibody reaction with an expression factor that is localized in cytoplasm of each histological type, and an antibody that shows an antigen-antibody reaction with an expression factor that is localized in a cell nucleus. The present invention provides improved tumor marker suitable for determining the histological type of lung carcinoma, by mixing an antibody that specifically shows an antigen-antibody reaction with a tumor marker that is localized in the cytoplasm with an antibody that specifically shows an antigen-antibody reaction with a tumor marker that is localized in the cell nucleus.

More specifically, the cocktail antibody of the present invention is used to determine the histological type of lung carcinoma, wherein an ADC cocktail antibody that shows an antigen-antibody reaction with Napsin-A that is localized in cytoplasm of adenocarcinoma and TTF-1 that is localized in a cell nucleus of such adenocarcinoma; and an SCC cocktail antibody that shows an antigen-antibody reaction with CK14 that is localized in cytoplasm of squamous cell carcinoma and p63 that is localized in a cell nucleus of such squamous cell carcinoma can be combined and used.

The above cocktail antibody is referred to as the ADC cocktail antibody because it is useful as a marker that indicates adenocarcinoma (ADC).

Also, the cocktail antibody is referred to as the SCC cocktail antibody because it is useful as a marker that indicates squamous cell carcinoma (SCC).

A kit for determining the histological type of non-small cell lung carcinoma may include the ADC cocktail antibody, the SCC cocktail antibody, and a reagent for performing immunohistochemical analysis using those antibodies, and may further include a control to determine adenocarcinoma, and a control to determine squamous cell carcinoma.

A method of determining the histological type of cancer tumor of the present invention is, a method of determining non-small cell lung carcinoma using the above cocktail antibody, wherein the method determines that a tumor that has been determined to be positive for the ADC cocktail antibody is adenocarcinoma, and determines that a tumor that has been determined to be positive for the SCC cocktail antibody is squamous cell carcinoma.

Furthermore, a tumor that has been determined to be negative for the ADC cocktail antibody and the SCC cocktail antibody may be subjected to immunohistochemical analysis using CK7, and a tumor has been determined to be positive may be determined to be adenocarcinoma.

The determination process is described in detail below. When using an antibody cocktail (ADC cocktail antibody) for each of TTF-1 and Napsin-A for adenocarcinoma, adenocarcinoma is recognized as a tumor that is either TTF-1 positive (nuclear positive) or Napsin-A positive (diffusely positive in cytoplasm). In all cases, squamous cell carcinoma is negative when subjected to this immunohistochemical analysis (hereinafter referred to as "immunostaining").

When using a cocktail antibody (SCC cocktail antibody) against p63 and cytokeratin 14, squamous cell carcinoma is recognized as a tumor with p63 immunostaining (diffusely positive in nuclear) and cytokeratin immunostaining (cytoplasmic positive).

Adenocarcinoma does not show immunostaining with the cocktail antibody to p63 and cytokeratin 14.

The present invention is described in detail below.

The antibodies used for the cocktail antibody according to the present invention are antibodies to TTF-1, Napsin-A, p63, and cytokeratin 14, respectively. The type (e.g., IgG or IgM) and the origin (e.g., mouse, rabbit, goat, or sheep) are not particularly limited.

Among the above, it is preferable to use a monoclonal antibody. Note that an oligoclonal antibody (mixture of several to several tens of types of antibodies) or a polyclonal antibody may also be used insofar as TTF-1, Napsin-A, p63, and cytokeratin 14 can be detected with sufficient specificity.

It is also possible to use an antibody fragment (e.g., Fab, Fab', F(ab')2, scFv, or dsFv or the like).

The above antibodies may be a commercially available product, or may be prepared by a known method such as an immunological method or a phage display method.

When determining (discriminating) squamous cell carcinoma and adenocarcinoma using the cocktail antibody according to the present invention, a sample may be prepared using cells/tissues collected from a living body, and subjected to an immunohistochemical method that is normally used for pathological diagnosis.

For example, the sample may be a thin section obtained by cutting a paraffin block in which cells/tissues collected from a living body are embedded, a cell/tissue microarray sheet prepared from a paraffin block in which cells/tissues collected from a living body are embedded, or the like.

Immunostaining used for the present invention may be performed by a immunohistochemical method (e.g., sampling→sample preparation→antigen-antibody reaction→visualization).

It is preferable to implement an antigen-antibody reaction and visualization using an enzyme-labeled antibody technique.

A standard protocol of the enzyme-labeled antibody technique is well-known in the art (see "Enzyme-Labeled Antibody Technique, Third Revised Edition", edited by Keiichi Watanabe and Kazuo Nakane, Gakusai Kikaku, for example).

An example of an immunostaining procedure for immunohistochemistry is described below.

### (1) Immobilization and paraffin embedding

Tissue collected from a living body (dead body in the case of autopsy) is immobilized using formalin, paraformaldehyde, or the like.

The tissue is then embedded in paraffin.

Tissue is normally dehydrated by using an alcohol, then treated with xylene, and finally embedded in paraffin.

The sample embedded in paraffin is cut to a desired thickness (e.g., 3 to 5 µm), and spread on a slide glass.

Note that a frozen sample may be used instead of a sample embedded in paraffin.

### (2) Deparaffinization

The sample is normally treated with xylene, an alcohol, and purified water in this order.

### (3) Pretreatment (antigen activation)

An enzyme treatment, a heat treatment, and/or a pressure treatment is optionally performed for antigen activation.

### (4) Removal of endogenous peroxidase

When using a peroxidase as a labeling substance for staining, endogenous peroxidase activity is blocked by treatment with a hydrogen peroxide solution.

### (5) Inhibition of non-specific reaction

The section is treated with a bovine serum albumin solution (e.g., 1% solution) for several to several tens of minutes to inhibit a non-specific reaction.

This step may be omitted when implementing the subsequent primary antibody reaction using an antibody solution that contains bovine serum albumin.

### (6) Primary antibody reaction

An antibody diluted to an appropriate concentration is dropped onto the section placed on the slide glass, and allowed to react for several tens of minutes to several hours.

After completion of the reaction, the section is washed with an appropriate buffer (e.g., phosphate buffer).

### (7) Addition of labelling reagent

A peroxidase is generally used as a labelling substance.

A secondary antibody to which a peroxidase is bound is dropped onto the section placed on the slide glass, and allowed to react for several tens of minutes to several hours.

After completion of the reaction, the section is washed with an appropriate buffer (e.g., phosphate buffer).

### (8) Chromogenic reaction

DAB (3,3'-diaminobenzidine) is dissolved in a Tris buffer.

A hydrogen peroxide solution is then added to the solution.

The resulting solution for chromogenic reaction is allowed to permeate the section for several minutes (e.g., 5 minutes) to effect color development.

After color development, the section is then sufficiently washed with tap water to remove DAB.

### (9) Dehydration, clearing, and mounting

The section is dehydrated using an alcohol, cleared with xylene, and then mounted in a synthetic resin, glycerol, gum syrup, or the like.

An example of procedure for use of the cocktail antibody according to the present invention is described below.
(1) A cell/tissue section cut from a paraffin block or an array sheet (hereinafter referred to as "specimen") is provided.
(2) The specimen is subjected to immunostaining using the cocktail antibody to each of TTF-1 and Napsin-A.
(3) The immunostained specimen is observed, and a tumor that is TTF-1 positive (nuclear positive) and/or Napsin-A positive (cytoplasm diffuse positive) is determined to be adenocarcinoma.
(4) The specimen is then subjected to immunostaining using the cocktail antibody for each of p63 and cytokeratin 14.
(5) The immunostained specimen is observed, and a tumor that is p63 positive (i.e., the nucleus is diffusely stained) and cytokeratin 14 positive (i.e., the cytoplasm is stained) is determined to be adenocarcinoma.

Note that an antibody for cytokeratin 7 (CK7) (i.e., auxiliary adenocarcinoma marker) may also be used in a third step.

A kit that utilizes the method of the present invention includes the following cocktail antibodies.
(a) ADC cocktail antibody for each of TTF-1 and Napsin-A
(b) SCC cocktail antibody for each of p63 and cytokeratin 14

The ADC cocktail and the SCC cocktail may be, for example, lyophilized, or a solution containing antibodies. It is preferable to use a solution from the viewpoint of convenience.

The kit may also include, for example, an antigen activation liquid, a washing solution, a blocking reagent, a buffer, a staining solution, a labeled secondary antibody, a reaction substrate, a control slide, and the like.

### EFFECTS OF THE INVENTION

The positive reactivity of p63 with squamous cell carcinoma is not 100%. Non-squamous epithelial NSCLC and squamous epithelial NSCLC can be descrimiated with an accuracy of about 100% by utilizing a cocktail antibody comprising an anti-p63 antibody and an anti-CK14 antibody which complement the anti-p63 antibody.

Moreover, an immunostaining method and determination for pathological diagnosis can be facilitated by the method according to the invention.

### BRIEF DESCRIPTION OF THE DRAWINGS

FIG 1 shows an immunostaining image of pathological samples of squamous cell carcinoma and adenocarcinoma with respect to tumor marker candidates.
FIG 2 shows an immunostaining method using a cocktail antibody and sensitivity of the method, and a method for evaluation of results.
FIG 3 is a scheme showing an algorithm for discrimination/screening for squamous cell carcinoma and adenocarcinoma.
FIG 4 shows the verification results for an ADC cocktail using a non-small cell lung carcinoma tissue array.
FIG 5 shows the verification results for an SCC cocktail using a non-small cell lung carcinoma tissue array.
FIG 6 shows the verification results (second step) for an SCC cocktail.
FIG 7 shows a case that is negative for an ADC cocktail and an SCC cocktail.

### BEST MODE FOR CARRYING OUT THE INVENTION

### Example 1

### 1. Selection of immunohistochemical marker specific to squamous cell carcinoma and adenocarcinoma of non-small cell lung carcinoma

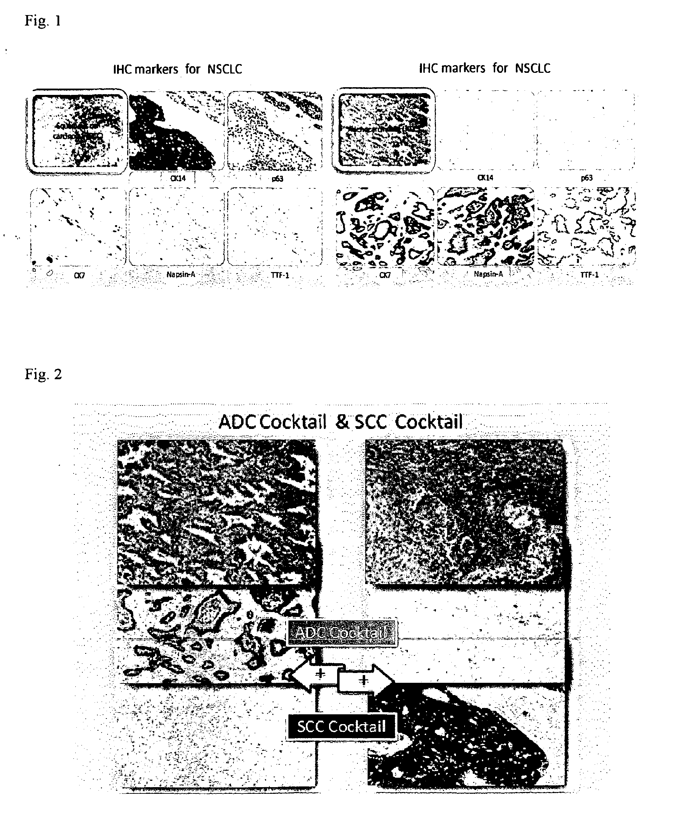

(1) Candidate markers shown in Table 1 were tested for squamous cell carcinoma (5 cases) and adenocarcinoma (7 cases), and cytokeratin 7 (CK7), cytokeratin 14 (CK14), Napsin-A, TTF-1, and p63 were selected as candidate markers for the cocktail antibody (see FIG 1).

**TABLE 1**

| Candidate marker | Location | Antibody | Clone | Manufacturer |
|---|---|---|---|---|
| CK5/6 | Cytoplasm | Mouse monoclonal | D5/16B4 | Dako |
| CK7 | Cytoplasm | Mouse monoclonal | K72 | Bio SB |
| CK8/18 | Cytoplasm | Mouse monoclonal | B22.1&B23.1 | Bio SB |
| CK14 | Cytoplasm | Mouse monoclonal | LL002 | Novocastra |
| CK17 | Cytoplasm | Mouse monoclonal | E3 | Novocastra |
| Napsin-A | Cytoplasm | Rabbit polyclonal | | IBL |
| TTF-1 | Cell nucleus | Mouse monoclonal | SPT24 | Novocastra |
| P63 | Cell nucleus | Mouse monoclonal | 4A4 | Dako |

Combination of a marker that is localized in cytoplasm and a marker that is localized in a cell nucleus were studied to make use of the specificity of each antibody and the complementary reactivity.

As shown in FIG 1, Napsin-A (localized in cytoplasm) and TTF-1 (localized in cell nucleus) were selected as a combination (ADC cocktail) of adenocarcinoma markers that were positive for adenocarcinoma and were negative for squamous cell carcinoma, and CK14 (localized in cytoplasm) and p63 (localized in cell nucleus) were selected as a combination (SCC cocktail) of squamous cell carcinoma markers that were negative for adenocarcinoma and were positive for squamous cell carcinoma.

### 2. Antibody cocktail for squamous cell carcinoma and antibody cocktail for adenocarcinoma

An antigen activation method, simplification of cumbersome treatment, and the concentration of each antibody and the mixing ratio were studied and optimized to make the best use of the specificity and the reactivity of each cocktail antibody candidate.

The most suitable immunostaining method and staining result evaluation method in terms of the sensitivity and the reactivity of the cocktail antibody were determined (see FIG 2).

### (1) Verification of immunostaining method using each cocktail antibody and confirmation of validity

A non-small cell lung carcinoma tissue array was prepared by choosing 15 cases of squamous cell carcinoma and 15 cases of adenocarcinoma diagnosed by the

Department of Pathology, Toyama University Hospital (see Table 2), and the cocktail antibody immunostaining method was verified using the tissue array.

In Table 2, the left half (column 0.000 to 6.000) indicates squamous cell carcinoma, and the right half (column 9.000 to 15.000) indicates adenocarcinoma.

**TABLE 2**

| | 0.000 | 3.000 | 6.000 | 9.000 | 12.000 | 15.000 |
|---|---|---|---|---|---|---|
| 0.000 | 03-0244-19 | 02-2468-17 | 00-2098-3 | 01-1518-5 | 01-1187-4 | 98-3836 |
| 3.000 | 03-0845-15 | 01-3517-15 | 00-1615-3 | 00-3062 | 00-3083-1 | 97-2803 |
| 6.000 | 03-1050-9 | 00-3577-1 | 00-0502-3 | 04-2931-10 | 02-2136-12 | 98-3350 |
| 9.000 | 02-0213-2 | 00-3005-14 | 99-1679-3 | 03-2781-9 | 03-2835-7 | 98-2665 |
| 12.000 | 02-12724-14 | 00-2416 | 99-745 | 00-3363-4 | 03-06468 | 97-3353 |

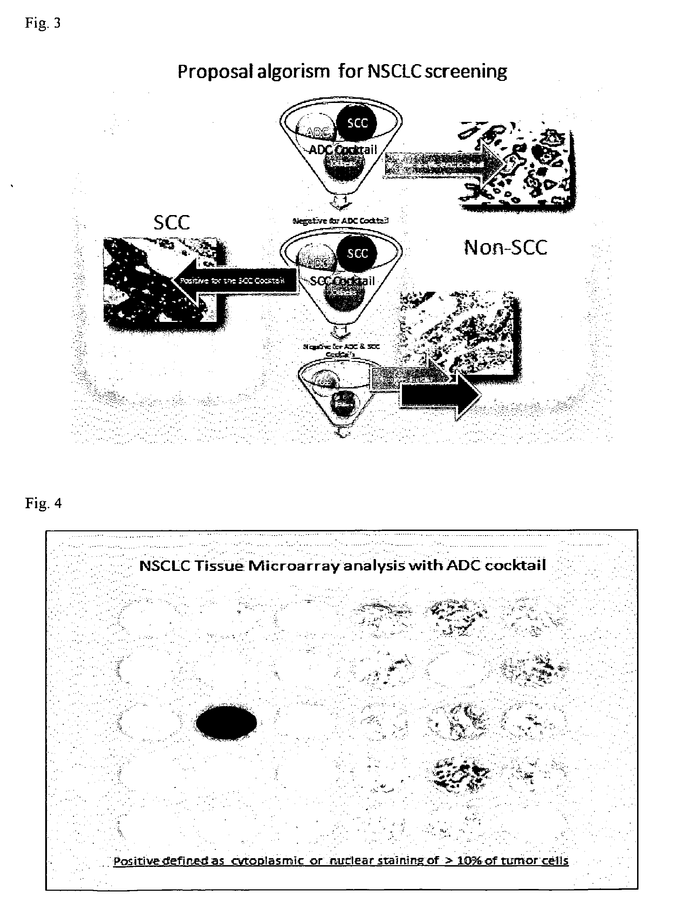

### (2) Algorithm for discrimination/screening squamous cell carcinoma and adenocarcinoma (FIG 3)

A first step of an algorithm for discrimination/screening squamous cell carcinoma and adenocarcinoma includes immunostaining using the ADC cocktail, and determining a case determined to be positive to be non-squamous cell carcinoma (i.e., adenocarcinoma). A second step of the algorithm includes subjecting the negative case to immunostaining using the SCC cocktail. A case determined to be positive in the second step is determined to be squamous cell carcinoma. A case determined to be negative using the ADC cocktail and the SCC cocktail is subjected to immunostaining using CK7 (auxiliary adenocarcinoma marker) (third step). A case determined to be positive in the third step is determined to be adenocarcinoma, and a case determined to be negative in the third step is determined to be other rare histological type of carcinoma. A case where 10% or more of the tumor cells were positively stained (cytoplasmic or nuclear staining) was determined to be positive.

### 3. Verification results for cocktail antibody using non-small cell lung carcinoma tissue array

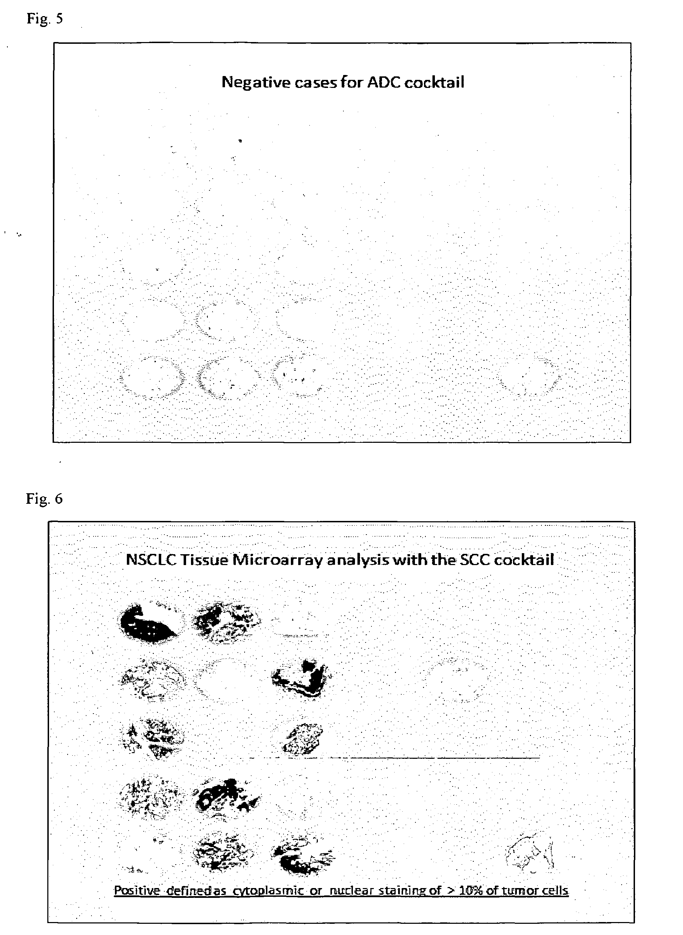

(1) In the first step (reaction with the ADC cocktail), for 29 cases (one case was not taken into consideration since a sufficient amount of tumor cells were not collected), 13 cases among the 15 adenocarcinoma cases (right half) were determined to be positive (FIG. 4).
   In FIG 4, 13 cases among the 15 adenocarcinoma cases (right half) were determined to be positive.
   All of the negative cases (16 cases (see FIG 5)) were subjected to immunostaining using the SCC cocktail.
   In FIG 5, 14 squamous cell carcinoma cases in the left half and 2 adenocarcinoma cases in the right half are negative.
(2) In the second step (reaction with the SCC cocktail), 14 cases among the 16 cases that were determined to be negative using the ADC cocktail were determined to be positive (FIG 6).

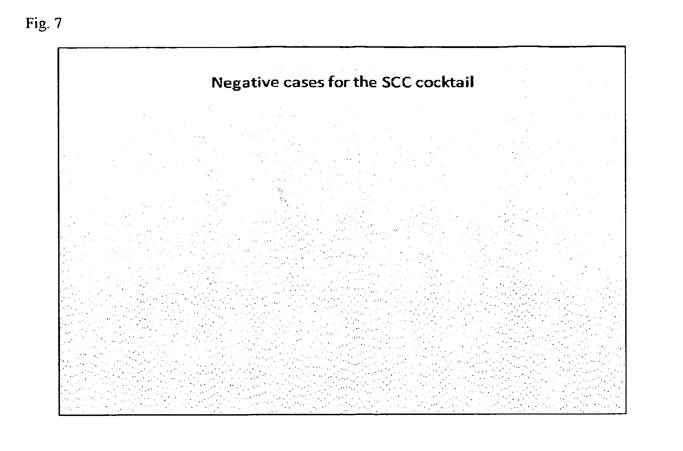

2 cases were determined to be negative using both of the ADC cocktail and the SCC cocktail (FIG. 7).

One of the 2 cases was determined to be obvious adenocarcinoma by morphological determination based on HE staining reexamination, and was also determined to be positive using CK7 (auxiliary adenocarcinoma marker).

### 4. Discussion of verification using tissue array

In discussing results of reaction by using ADC cocktail antibody and SCC cocktail antibody, 1 case (ID: 97-3353) among the 15 adenocarcinoma cases was negative for the ADC cocktail antibody and positive for the SCC cocktail antibody.

1 case (ID: 01-3517-15) among the 14 squamous cell carcinoma cases was negative for the ADC cocktail antibody and the SCC antibody.

These cases were reexamined based on the reactivity with other markers and HE staining. As a result, the case 97-3353 (previously diagnosed as adenocarcinoma) was determined to be a rare basaloid type of squamous cell carcinoma.

On the other hand, the case 01-3517-15 was positive for cytokeratin markers Pan-CK and CK8/18 and exhibited glandular epithelium origin, and determined to be large cell carcinoma based on the size of the tumor cells and the like as a result of reexamination of an HE staining morphological determination.

These cases were classified as squamous cell carcinoma and other carcinoma (large cell carcinoma), respectively (i.e., 14 adenocarcinoma cases, 14 squamous cell carcinoma cases, and 1 other carcinoma (large cell carcinoma) case (29 cases in total)).

Eventually, for the ADC cocktail antibody, the specificity to adenocarcinoma (14 cases) was 100%, and the sensitivity was 92%.

When using the SCC cocktail antibody for the 14 cases, the specificity was 100%, and the sensitivity was 100%.

### INDUSTRIAL APPLICABILITY

The immunohistochemical method using the ADC cocktail antibody and the SCC cocktail antibody according to the present invention exhibits excellent specificity and sensitivity when determining the histological type (squamous cell carcinoma or adenocarcinoma) of non-small cell lung carcinoma (NSCLC), and is very useful for pathological diagnosis.

## Claims

1. A cocktail antibody used for determining the histological type of lung carcinoma, the cocktail antibody comprising:
(a) an ADC cocktail antibody that shows an antigen-antibody reaction with Napsin-A localized in cytoplasm of adenocarcinoma and TTF-1 localized in a cell nucleus of adenocarcinoma; and
(b) an SCC cocktail antibody that shows an antigen-antibody reaction with CK14 localized in cytoplasm of squamous cell carcinoma and p63 localized in a cell nucleus of squamous cell carcinoma.

2. A kit for determining a histological type of non-small cell lung carcinoma, comprising an ADC cocktail antibody as defined in claim 1.(a), an SCC cocktail antibody as defined in claim 1.(b), and a reagent for performing immunohistochemical analysis using the antibodies.

3. The kit according to claim 2, further comprising:
(a) an adenocarcinoma determination control; and
(b) a squamous cell carcinoma determination control.

4. An in vitro method of determining the histological type of non-small cell lung carcinoma using the cocktail antibody according to claim 1, comprising:
(a) determining a tumor that has been determined to be positive for the ADC cocktail antibody as being adenocarcinoma; and
(b) determining a tumor that has been determined to be positive for the SCC cocktail antibody as being squamous cell carcinoma.

5. The method according to claim 4, further comprising:
(a) subjecting a tumor, that has been determined to be negative for the ADC cocktail antibody and negative for the SCC cocktail antibody, to immunohistochemical analysis of CK7, and
(b) determining the tumor to be adenocarcinoma when the tumor has been determined to be positive for CK7.

## Patentansprüche

1. Cocktail-Antikörper zur Bestimmung des histologischen Typs eines Lungenkarzinoms, wobei der Cocktail-Antikörper folgendes umfasst:
(a) einen ADC-Cocktail-Antikörper, der eine Antigen-Antikörper-Reaktion mit im Cytoplasma eines Adenokarzinoms lokalisiertem Napsin-A und mit in einem Zell-Nukleus eines Adenokarzinoms lokalisiertem TTF-1 zeigt; und
(b) einen SCC-Cocktail-Antikörper, der eine Antigen-Antikörper-Reaktion mit im Cytoplasma eines Plattenepithelkarzinoms lokalisiertem CK14 und mit in einem Zell-Nukleus eines Plattenepithelkarzinoms lokalisiertem p63 zeigt.

2. Kit zur Bestimmung eines histologischen Typs eines nicht-kleinzelligen Lungenkarzinoms, umfassend einen wie in Anspruch 1(a) definierten ADC-Cocktail-Antikörper, einen wie in Anspruch 1(b) definierten SCC-Cocktail-Antikörper und ein Reagenz für die Durchführung einer immunhistologischen Analyse unter Verwendung der Antikörper.

3. Kit nach Anspruch 2, ferner umfassend:
(a) eine Kontrolle für die Bestimmung eines Adenokarzinoms; und
(b) eine Kontrolle für die Bestimmung eines Plattenepithelkarzinoms.

4. *In vitro* Verfahren zur Bestimmung des histologischen Typs eines nicht-kleinzelligen Lungenzellkarzinoms unter Verwendung der Cocktail-Antikörper nach Anspruch 1, bei dem man:
(a) einen Tumor, der als positiv für den ADC-Cocktail-Antikörper nachgewiesen wurde, als Adenokarzinom bestimmt; und
(b) einen Tumor, der als positiv für den SCC-Cocktail-Antikörper nachgewiesen wurde, als Plattenepithelkarzinom bestimmt .

5. Verfahren nach Anspruch 4, bei dem man ferner:
(a) einen Tumor, der als negativ für den ADC-Cocktail-Antikörper und als negativ für den SCC-Cocktail-Antikörper bestimmt wurde, einer immunohistochemischen Analyse in Bezug auf CK7 unterzieht, und
(b) den Tumor als Adenokarzinom nachweist, wenn der Tumor als positiv für CK7 bestimmt wurde.

## Revendications

1. Anticorps en cocktail utilisé pour déterminer le type histologique d'un carcinome du poumon, l'anticorps en cocktail comprenant :
(a) un anticorps en cocktail d'ADC qui présente une réaction antigène-anticorps avec la Napsine-A localisée dans le cytoplasme d'un adénocarcinome et le TTF-1 localisé dans un noyau cellulaire d'un adénocarcinome ; et
(b) un anticorps en cocktail de SCC qui présente une réaction antigène-anticorps avec la CK14 localisée dans le cytoplasme d'un carcinome à cellules squameuses et la p63 localisée dans un noyau cellulaire d'un carcinome à cellules squameuses.

2. Kit permettant de déterminer un type histologique d'un carcinome du poumon non à petites cellules, comprenant un anticorps en cocktail d'ADC tel que défini dans la revendication 1.(a), un anticorps en cocktail de SCC tel que défini dans la revendication 1.(b), et un réactif pour effectuer une analyse immunohistochimique en utilisant les anticorps.

3. Kit selon la revendication 2, comprenant en outre :
(a) un témoin de détermination d'adénocarcinome ; et
(b) un témoin de détermination de carcinome à cellules squameuses.

4. Procédé *in vitro* de détermination du type histologique d'un carcinome du poumon non à petites cellules en utilisant l'anticorps en cocktail selon la revendication 1, comprenant le fait :
(a) de déterminer une tumeur, qui a été déterminée comme étant positive pour l'anticorps en cocktail d'ADC, comme étant un adénocarcinome ; et
(b) de déterminer une tumeur, qui a été déterminée comme étant positive pour l'anticorps en cocktail de SCC, comme étant un carcinome à cellules squameuses.

5. Procédé selon la revendication 4, comprenant en outre le fait :
(a) de soumettre une tumeur, qui a été déterminée comme étant négative pour l'anticorps en cocktail d'ADC et négative pour l'anticorps en cocktail de SCC, à une analyse immunohistochimique de CK7, et
(b) de déterminer la tumeur comme étant un adénocarcinome lorsque la tumeur a été déterminée comme étant positive pour la CK7.
